Europäisches Patentamt

European Patent Office

Office européen des brevets

(11) Veröffentlichungsnummer: **0 229 398 B1**

(19)

(12) **EUROPÄISCHE PATENTSCHRIFT**

(45) Veröffentlichungstag der Patentschrift: **12.02.92**

(21) Anmeldenummer: **86118157.6**

(22) Anmeldetag: **30.12.86**

(51) Int. Cl.⁵: **A61J 3/07**, **A61K 9/48**

(54) **Kapseln sowie Verfahren und Vorrichtung zur Herstellung solcher Kapseln.**

(30) Priorität: **03.01.86 DE 3600084**

(43) Veröffentlichungstag der Anmeldung:
**22.07.87 Patentblatt 87/30**

(45) Bekanntmachung des Hinweises auf die
Patenterteilung:
**12.02.92 Patentblatt 92/07**

(84) Benannte Vertragsstaaten:
**AT BE CH DE ES FR GB GR IT LI NL SE**

(56) Entgegenhaltungen:
**CH-A- 267 221**
**DD-A- 201 643**
**DE-A- 2 735 393**
**US-A- 3 435 110**

(73) Patentinhaber: **R.P. Scherer GmbH**
**Gammelsbacher Strasse 2 Postfach 1243**
**W-6930 Eberbach/Baden(DE)**

(72) Erfinder: **Schönmann, Holger, Dr.**
**Gerhart-Hauptmann-Strasse 15**
**W-6930 Eberbach(DE)**
Erfinder: **Eck, Hans-Peter**
**Pfarrhof 2**
**W-6930 Eberbach(DE)**

(74) Vertreter: **Lehmann, Klaus, Dipl.-Ing. et al**
**Patentanwälte Schroeter & Lehmann Post-**
**fach 71 03 50 Wolfratshauser Strasse 145**
**W-8000 München 71(DE)**

EP 0 229 398 B1

## Beschreibung

Die Erfindung betrifft ein Verfahren zur Hertellung eines Formkörpers der mit einer Hülle aus Polymer-Material, Gelatine oder dergleichen, und mit einer Füllung in der Hülle versehen ist. Im allgemeinen handelt es sich hierbei um harte oder weiche Gelatinekapseln, die mit pharmazeutischen Präparaten gefüllt sind. Die Erfindung betrifft auch den Formkörper sowie eine entsprechende zur Herstellung der Formkörper bzw. zur Durchführung des Verfahrens geeignete Vorrichtung.

Für die Herstellung von Weichgelatinekapseln hat sich im wesentlichen das sogenannte Rotary-Die-Verfahren durchgesetzt, bei dem Gelatinebänder über mit hohlen Formvertiefungen versehene Formwalzen geführt und zwischen diese eingeführt werden. Mit Hilfe eines Füllkeils werden einerseits die Gelatinebänder in die Formvertiefungen hineingedrückt, und andererseits wird gleichzeitig das Füllgut in die so gebildeten Kapseln eingespritzt. Die Weichkapseln werden also in einem Arbeitsgang hergestellt und befüllt. Dabei kann jeweils nur ein einheitlicher Stoff oder eine homogene Stoffmischung, also eine Mischung aus mehreren Substanzen, abgefüllt werden.

Daneben sind Hartgelatinekapseln bekannt, die aus zwei vorgefertigten Kapselteilen teleskopartig zusammengesteckt werden. Vor dem Zusammenstecken werden in den Kapselhauptteil in einem besonderen Arbeitsgang eine einheitliche Substanz oder eine homogene Stoffmischung oder in mehreren Arbeitsschritten nacheinander verschiedene Lagen von Stoffen oder Stoffgemischen abgefüllt. Die Herstellung solcher Steckkapseln ist daher technisch aufwendiger und kostspieliger als die Herstellung von Weichgelatinekapseln. Bei Hartgelatinekapseln muß bei Verwendung mehrerer verschiedener Lagen von Füllstoffen dafür Sorge getragen werden, daß die Stoffe miteinander kompatibel sind und daß eine Durchmischung der Stoffe nach dem Füllen zulässig ist. Siehe auch das in CH-A-267221 beschriebene Verfahren.

Der Erfindung liegt die Aufgabe zugrunde, ein Herstellungsverfahren einen Formkörper, sowie eine Herstellungsvorrichtung hierfür zu schaffen, bei denen es möglich ist, verschiedene Substanzen oder Stoffgemische, und zwar auch solche, die miteinander nicht kompatibel sind oder die miteinander vorzeitig reagieren würden, in einem Arbeitsgang in den gleichen Formkörper abzufüllen, so daß innerhalb des gleichen Formkörpers dauerhaft unterschiedliche Stoffzonen vorhanden sind.

Demgemäß besteht erfindungsgemäß das Verfahren zur Herstellung eines solchen Formkörpers darin, daß in verschiedene Bereiche einer gemeinsamen Hülle gleichzeitig mindestens zwei Wirkstoffe mit ihren zugehörigen Trägerstoffen ohne Trennschicht eingebracht werden (Patentanspruch 1).

Der erfindungsgemäße Formkörper ist dadurch gekennzeichnet, daß die Trägerstoffe (Phasen) nebeneinander geschichtet ohne Trennwand in der Hülle angeordnet sind (Patentanspruch 5).

Durch die Erfindung werden also ein Formkörper sowie ein dafür geeignetes Herstellungsverfahren geschaffen, bei denen es durch gleichzeitiges Abfüllen mehrerer Substanzen möglich ist, in einen einzigen Kapselformkörper zwei oder mehr geschichtete, gleiche oder unterschiedliche Phasen einzubringen. Hiermit ist es einerseits möglich, gleiche Wirkstoffe in verschiedenartige Trägerstoffe oder Matrices eingebettet so in der gleichen Hülle als Dosiereinheit unterzubringen, daß z.B. bei pharmazeutischen Produkten eine unterschiedliche Resorptionsgeschwindigkeit im Körper erreicht wird. Andererseits ist es möglich, verschiedenartige Wirkstoffe, die an sich nicht miteinander kompatibel sind oder die sich in unerwünschter Weise miteinander vermischen würden, ohne gegenseitige Reaktion oder Vermischung mit Hilfe gleicher oder unterschiedlicher Trägerstoffe oder Phasen nebeneinander ohne Trennwand in einer gemeinsamen Hülle unterzubringen. Dabei bilden die Trägerstoffe oder Phasen jeweils eine stabile Matrix in ihrem zugehörigen Teilbereich innerhalb der Hülle, wobei auch eine Vernetzung der Trägerstoffe oder ein Erstarren von flüssig eingebrachten Füllungen innerhalb der Hülle möglich ist. Die Trägerstoffe können auch mit dem Material der Hüllwand, also beispielsweise mit der Gelatine vernetzen. Prinzipiell kann es sich um gleiche oder gleichartige Trägerstoffe handeln, sofern nur sichergestellt ist, daß ein Austausch oder eine Reaktion oder Wechselwirkung zwischen den Wirkstoffen in den verschiedenen Bereichen ausgeschlossen ist bzw. eine Diffusion an den Trennflächen zwischen den verschiedenen Bereichen nur sehr langsam oder über lange Zeiträume erfolgt.

Durch die Abfüllung der Substanzen in getrennte Bereiche eines einheitlichen Formkörpers lassen sich z.B. folgende Vorteile erreichen:

Bei Arzneimitteln erfolgt gemäß Anspruch 3 eine chemische Stabilisierung unterschiedlicher, miteinander inkompatibler Wirkstoffe, die bei Berührung miteinander reagieren würden, derart, daß sie in der gleichen Dosiereinheit praktisch beliebig aufbewahrt werden können und eine Wechselwirkung der reaktionsfähigen Stoffe bis zum Zeitpunkt der Einnahme des Formkörpers durch einen Patienten vermieden ist. Erst nach der Einnahme und der Auflösung der Hülle können die Wirkstoffe dann zu einer Wechselwirkung miteinander kommen.

Bei einem Formkörper mit den Merkmalen von Anspruch 2 kann man eine unterschiedliche Resorption gleicher oder unterschiedlicher Stoffe aus dem gleichen Formkörper erreichen, wobei z.B. die Wirkstoffe im Körper des Patienten zeitlich nacheinander an gleichen oder verschiedenen Stellen zur Wirkung kommen.

Wenn nach der Herstellung des gefüllten Formkörpers in diesem eine bestimmte Matrix ausgebildet wird, kann durch Wechselwirkung verschiedener Matrices nachträglich noch ein retardierend wirkender Kapselinhalt ausgebildet werden. So können beim Abfüllen von Wirkstoffen, die mit dem Material, beispielsweise der Gelatine, der Hülle vernetzend reagieren, innerhalb einer durch den Formkörper gebildeten Einzeldosis Bereiche gebildet werden, die nicht miteinander vernetzen, so daß beim Zerfall, Öffnen oder Aufschließen der Hülle im Körper des Patienten nach einer bestimmten Zeit oder innerhalb einer vorbestimmten Zeit unterschiedliche Teildosen gewährleistet bleiben, die besondere therapeutische Wirkungen haben können. Dabei können innerhalb des gleichen Formkörpers Bereiche unterschiedlicher Löslichkeit ausgebildet werden, z.B. auch dadurch, daß Teile der Hülle in Wasser oder Körperflüssigkeit löslich und andere Teile der Hülle in den gleichen Medien schwer oder nicht löslich sind (Anspruch 6), wodurch die Wechselwirkungsmöglichkeiten zwischen Hülle und/oder Wirkstoff und/oder Trägerstoff vervielfältigt werden. Insgesamt erreicht man eine Verbesserung der Darreichungsmöglichkeiten für Medikamente, wobei physikalische oder chemische Unverträglichkeiten der Wirkstoffe gezielt und getrennt beherrscht werden können. Die Wirkstoffe können in dem gemeinsamen Formkörper nach dem Abfüllen auch ähnlich wie in einem Schwamm enthalten sein und daraus nur langsam abgegeben werden.

Die die jeweilige Matrix bildenden Trägerstoffe können lipophil sein, also beispielsweise aus Ölen, Wachsen oder Fetten bestehen, mit denen die Wirkstoffe mischfähig oder in denen sie lösbar sind. Die Trägerstoffe können auch hydrophil sein, also auf Wassergrundlage gebildet sein, wie beispielsweise Polyglykole, wobei die Wirkstoffe in diesen Medien vermischt, suspendiert oder gelöst sind.

Die erfindungsgemäße Vorrichtung zur Herstellung der beschriebenen Formkörper bzw. zur Durchführung des Herstellungsverfahrens erfordert gegenüber bekannten Kapselherstellungsmaschinen nur geringfügige Änderungen. Im wesentlichen benötigt man statt bisher einer Station für die Zuführung des Füllmediums eine entsprechende Mehrzahl solcher Stationen. Bei einer Herstellungsmaschine für Weichkapseln, die nach dem sogenannten "Rotary-Die-Verfahren" arbeitet, werden also zwei oder mehr Zuführkanäle für das Füllmedium mit einer entsprechenden Zahl von Einspritzdüsen vorgesehen, die in ein und denselben Formhohlraum münden, der aus den beiden Formhälften der beiden Formwalzen besteht. Daneben ist es nur erforderlich, den sehr präzise arbeitenden Zuführmechanismus so umzugestalten, daß das Abfüllen verschiedener Lösungen oder Mischungen auch bei unterschiedlicher Viskosität noch präzise möglich ist. Hiermit können nicht nur gleiche, sondern auch unterschiedliche Füllstoffe (gleiche und/oder unterschiedliche Wirkstoffe, gleiche und/oder unterschiedliche Trägersubstanzen) zugeführt werden. Damit lassen sich z.B. auch normalerweise nicht miteinander mischbare Bestandteile, die man bisher nicht in einem einzigen Arbeitsgang abfüllen konnte, genau dosiert in einen einheitlichen Formkörper ohne Trennwand einbringen. Bisher war es nach dem Stand der Technik nur möglich, vorgemischte homogene Substanzen dosiert in einen Formkörper abzufüllen, wobei darauf geachtet werden mußte, daß die Substanzen nicht schon beim Zuführen oder beim Abfüllen oder bei der Lagerung im Formkörper miteinander reagierten. Hierbei war es bisher auch nicht möglich, Wirkstoffe nacheinander im Körper eines Patienten wirksam werden zu lassen. In vielen Fällen trat eine Reaktion der verschiedenen Stoffe schon im Vorratsbehälter oder in der Zuführleitung vor der Füllstation ein, so daß eine Abfüllung bestimmter Stoffe überhaupt nicht möglich war. Durch die getrennte Zuführung und das getrennte, jedoch gleichzeitige Einbringen in einen einheitlichen Formkörper lassen sich diese Probleme vermeiden.

**Patentansprüche**

1. Verfahren zum Herstellen einer Kapsel, insbesondere einer Gelatinekapsel, zur Aufnahme von pharmazeutischen Wirkstoffen, **dadurch gekennzeichnet**,
daß in verschiedene Bereiche einer gemeinsamen Hülle gleichzeitig mindestens zwei Wirkstoffe mit ihren zugehörigen Trägerstoffen ohne Trennschicht eingebracht werden.

2. Verfahren nach Anspruch 1, **dadurch gekennzeichnet**,
daß die Wirkstoffe vor dem Einbringen in Trägerstoffe eingebracht werden, die untereinander nicht mischbar sind.

3. Verfahren nach einem der vorhergehenden Ansprüche,
**dadurch gekennzeichnet**,
daß die Trägerstoffe lipophil oder hydrophil sind.

4. Verfahren nach einem der vorhergehenden Ansprüche,
**dadurch gekennzeichnet**,

daß die Wirkstoffe mit ihren Trägerstoffen gleichzeitig durch mindestens zwei Kanäle in eine gemeinsame Kapsel eingespritzt werden.

5. Kapsel zur Aufnahme von Wirkstoffen, die in verschiedenen Bereichen einer gemeinsamen Hülle gleichzeitig mindestens zwei Wirkstoffe mit ihren zugehörigen Trägerstoffen enthält, erhältlich nach einem Verfahren nach einem der Ansprüche 1 bis 4, **dadurch gekennzeichnet**, daß
die Hülle aus Gelatine, insbesondere Weichgelatine, besteht, und daß die Trägerstoffe nebeneinander geschichtet ohne Trennwand in der Hülle augeordnet sind.

6. Kapsel zur Aufnahme von Wirkstoffen, nach Anspruch 5 **dadurch gekennzeichnet**,
daß Teile der Hülle in Wasser oder Körperflüssigkeiten löslich und andere Teile der Hülle in den gleichen Medien schwer oder nicht löslich sind.

7. Vorrichtung zur Durchführung des Verfahrens nach einem der Ansprüche 1 bis 4 oder zur Herstellung einer Kapsel nach Anspruch 5 oder 6, wobei die Vorrichtung zwei Formwalzen mit zusammenwirkenden Formvertiefungen, die jeweils einen Formhohlraum bilden, und einen Füllkeil zum Füllen der Kapsel mit Wirkstoffen aufweist, **dadurch gekennzeichnet,** daß im Füllkeil mindestens zwei in den Bereich eines Formhohlraums mündende Zuführkanäle zum gleichzeitigen Abfüllen einer entsprechenden Zahl von Trägerstoffen mit den zugehörigen Wirkstoffen in eine Kapsel vorgesehen sind.

**Claims**

1. A method for manufacturing a capsule, particularly a gelatine capsule, for receiving pharmaceutical active ingredients, **characterized in that** into different regions of a common enclosure at the same time at least two active ingredients with their associate carrier substances are introduced without a partition.

2. Method according to claim 1, **characterized in that** the active ingredients before their introduction are introduced into carrier substances which are not mixable with each other.

3. Method according to one of the preceding claims, **characterized in that** the carrier substances are lipophilous or hydrophilous.

4. Method according to one of the preceding claims, **characterized in that** the active ingredients with their carrier substances are simultaneously injected through at least two channels into a common capsule.

5. Capsule for receiving active ingredients, which in different regions of a common enclosure simultaneously contains at least two active ingredients with their associate carrier substances, obtainable by a method according to one of the claims 1 to 4, **characterized in that** the enclosure consists of gelatine, particularly soft gelatine, and that the carrier substances are layered besides each other without a partition in the enclosure.

6. Capsule for receiving active ingredients according to claim 5, **characterized in that** parts of the enclosure are soluble in water or body fluids and other parts of the enclosure are scantily soluble or insoluble in the same media.

7. Apparatus for carrying out the method according to one of the claims 1 to 4 or for manufacturing a capsule according to claim 5 or 6, wherein the apparatus comprises two forming rollers with cooperating mold recesses each forming a mold cavity, and a filling wedge for filling the capsule with active ingredients, **characterized in that** in the filling wedge there are provided at least two supply conduits terminating in the region of one mold cavity for simultaneously filling a corresponding number of carrier substances with the associate active ingredients into one capsule.

**Revendications**

1. Procédé de fabrication d'une capsule, en particulier d'une capsule en gélatine, pour recevoir des substances actives pharmaceutiques, **caractérisé** en ce que, dans des régions différentes d'une enveloppe commune, au moins deux substances actives sont introduites simultanément avec leurs substances porteuses afférentes, sans couche de séparation.

2. Procédé selon la revendication 1, caractérisé en ce que les substances actives sont introduites dans des substances porteuses non miscibles entre elles, avant l'introduction.

3. Procédé selon une des revendication précédentes, caractérisé en ce que les substances porteuses sont lipophiles ou hydrophiles.

4. Procédé selon une des revendications précédentes, caractérisé en ce que les substances actives sont injectées simultanément avec leurs substances porteuses par au moins deux canaux dans une capsule commune.

5. Capsule pour recevoir des substances actives, contenant simultanément dans des zones différentes d'une enveloppe commune deux substances actives avec leurs substances porteuses afférentes, cette capsule pouvant être obtenue par un procédé selon une des revendications 1 à 4, caractérisé en ce que l'enveloppe est en gélatine, en particulier en gélatine souple, et en ce que les substances porteuses sont disposées dans l'enveloppe en couches l'une à côté de l'autre, sans paroi de séparation.

6. Capsule pour recevoir des substances actives, selon la revendication 5, caractérisée en ce que des parties de l'enveloppe sont solubles dans l'eau ou dans les fluides corporels et d'autres parties de l'enveloppe sont difficilement solubles ou insolubles dans ces mêmes fluides.

7. Dispositif pour la mise en oeuvre du procédé selon une des revendications 1 à 4 ou pour la fabrication d'une capsule selon la revendication 5 ou 6, ce dispositif présentant deux cylindres de formage avec des creux de formage coopérant ensemble qui constituent chaque fois une cavité de formage, et un coin de remplissage pour le remplissage de la capsule avec des substances actives, **caractérisé** en ce que dans le coin de remplissage sont prévus au moins deux canaux d'alimentation débouchant dans la zone d'une cavité de formage, pour l'introduction simultanée d'un nombre correspondant de substances porteuses avec les substances actives associées, dans une capsule.